# EUROPEAN PATENT SPECIFICATION

(11) **EP 4 247 314 B1**
(45) Date of publication and mention of the grant of the patent: **27.11.2024**
(21) Application number: 21814939.1
(22) Date of filing: 01.10.2021
(51) Int. Cl.: A61F 13/20

(54) **SHAPE RETAINING, PHYSIOLOGICALLY ACCEPTABLE AND BIOLOGICALLY DEGRADABLE HYGIENIC TAMPON AND THE PROCESS FOR MANUFACTURING SUCH A TAMPON**
FORMSTABILER, PHYSIOLOGISCH AKZEPTABLER UND BIOLOGISCH ABBAUBARER HYGIENETAMPON UND VERFAHREN ZUR HERSTELLUNG SOLCH EINES TAMPONS
TAMPON À MAINTIEN DE FORME, PHYSIOLOGIQUEMENT ACCEPTABLE ET BIOLOGIQUEMENT DÉGRADABLE, ET PROCÉDÉ DE FABRICATION D'UN TEL TAMPON

(30) Priority: 13.10.2020 SI 202000186
(43) Date of publication of application: 27.09.2023
(73) Proprietor: TOSAMA Tovarna sanitetnega materiala d.o.o., 1230 Domzale (SI)
(72) Inventor: KOVAC, Gregor, 1251 Moravce (SI); BRDNIK, Tomaz, 1293 Smarje Sap (SI); JERIN, Franci, 1230 Domzale (SI)
(74) Representative: Borstar, Dusan
(86) International application number: PCT/SI2021/000009
(87) International publication number: WO 2022/081096

(56) References cited:
- EP-B1- 2 629 726
- WO-A2-02/076357
- US-A- 4 787 895
- US-A1- 2008 097 366

## Description

The disclosure refers to a tampon, especially a hygienic tampon. Pursuant to the International Patent Classification such inventions in the area of common life necessities belong to medicine and hygiene, namely to tampons, in particular menstrual hygienic tampons and accessories therefor, and therefore corresponds to class A 61 F 13/20.

The invention is based on a problem, how to assure a regular and at least approximately cylindrical shape of a tampon both in its dry state prior to use and also in its moisturized state after the use thereof, and in particularly also to prevent unwinding of layers of moisturized fibers from the surface of the tampon, which might lead to transforming of such tampon into a substantially asymmetric shape and during the extraction of such tampon even to retaining of parts of the tampon within said body cavity, wherein such tampon must be suitable for inserting into a body cavity and needs also be wholly biologically degradable within a reasonable time period after the use and deposition to the waste, and wherein such tampon should also be manufactured by using functionally comparable raw materials and preferably the same machines suitable for performing of substantially the same manufacturing steps as by known and commercially available tampons of similar art, which could then lead to a required absorption capacity of the tampon and also to safety and reliability during the use thereof, and moreover also the attention should be paid to maintaining of a physiological applicability in view of preventing potential irritations and inflammations of exposed human body areas, especially the mucosa, which might be generally caused due to contacting such tampon and long-term exposure to artificial materials during the use of the tampon.

Especially in view of the shape and technical characteristics of the tampon, a comparable prior art tampon is disclosed in EP 0 422 660. The described tampon consists of an unwoven fibrous blank, which is obtained by winding an unwoven fibrous band around the longitudinal axis, which results in an at least approximately cylindrical blank, which is then radially compressed by means of suitable jaws, for example by using a compressing means as described in DE 19 825 877, formed into a tampon. Such a tampon is principally still of at least approximately cylindrical shape, but is furnished with grooves, which are impressed on its surface and are each per se directed radially inwards, while at the same time said grooves extend along said external surface in the longitudinal direction of the tampon, namely in parallel with said longitudinal i.e. central axis of the tampon. An apparatus and a process for manufacturing such tampons are generally described in WO 85/02996 A1.

Such tampons are known to those skilled in the art as »digital« tampons. By further modifying the tampon, namely by suitably shaping the front end portion i.e. the head of the tampon, such tampon may then also be used as an »applicator« tampon, which is e.g. together with a related applicator disclosed in WO 2011/115586 A1.

Between each two adjacent grooves there is a rib, wherein the outer surfaces of all ribs along the circumference determines the outer diameter of the tampon, while the bottom of said grooves determines the diameter of a relatively compact tampon core. By means of a subsequent, relatively smooth radially compression in the area of said ribs, certain convexity, i.e. deflection of lateral surfaces of said ribs can be achieved, so that the lateral surfaces of each adjacent ribs remain in contact with each other and in the adjacency of said core form a tubular space, which extends parallel to the longitudinal axis of the tampon. Such a design may lead to positive effects, namely to increased bending and buckling strength of the tampon and in particular also increases specific absorption capacity in the longitudinal direction of the tampon. The stability of the tampon is of decisive significance from the perspective of trust by the user and is especially important during the insertion of the tampon. During insertion it is also important that, in addition to said bending and buckling stability, the external surface of the tampon, which is during use in direct contact with the mucosa, should be as smooth as possible, so that the friction coefficient on the external surface of the tampon is as small as possible.

For this purpose, the known tampon is on its exterior surface surrounded with a liquid permeable wrapper, but its composition is different and especially more compact than the composition of the absorbent tampon core itself. The purpose of the wrapper is to ensure a pleasant feel and a suitable friction coefficient on the surface of the tampon, which makes insertion of the tampon into a body cavity easier. A further purpose of said wrapper envelope is to prevent individual fibers or the absorbent core of the tampon to be retained in the body cavity during the removal of said tampon.

In a process of manufacturing such tampon, a fibrous band of suitable width and length is prepared, across which a band of material forming said wrapper is placed, which is generally at least the same width and of such length, that it exceeds the circumference of such wrapped tampon blank prior to compression thereof. Said wrapper band is secured to the absorbent fibrous band by means of adhesive or by welding, and upon that at the a retaining string is placed transversally across the absorbent band, which is then followed by winding into a roll around the geometric axis, which passes transversally with regards to said absorbent fibrous band and wrapper band, which are therefore helically wound to form an at least approximately cylindrical blank, the external surface of which is completely surrounded by said wrapper and is furnished with a retaining string extending in approximately axial direction there-from. Subsequently, said blank undergoes compression by means of a pre-determined amount of jaws, which are arranged radially with regard to said blank and are equidistantly distributed around the entire circumference, wherein on one hand the diameter of the blank is essentially reduced, which leads to a significantly compressed absorbent core formed inside of the tampon, and on the other hand also to multiple grooves and ribs, which are formed on the surface of the tampon and are equidistantly distributed around the circumference, wherein surface is covered by said wrapper. Optionally, in the same step, or in an additional subsequent step, a front insertion area is properly formed, the a called head of the tampon is obtained, which can be formed in compliance with the specifications of the tampon type with regard to insertion, for the purposes of a digital or an applicator tampon. Regarding the tampon according to EP 0 422 660, after compression with the purpose of forming said grooves and ribs, during a subsequent step said ribs are exposed to additional external pressure, upon which the ribs become compacted and. deformed to such an extent, that they contact each other along the circumference of the tampon and that said grooves remain enclosed inside of the tampon, in the adjacency of said core. In such tampon according to EP 0 422 660, the silhouette of the tampon should be symmetrical and the depth of all grooves uniform along the circumference.

Tampons are also known in the state of the art, which have a symmetric silhouette, however the depth of a certain number of grooves is smaller than the depth of the other grooves. Such tampon is disclosed in EP 2 440 169 B1.

As mentioned, the tampon wrapper is liquid permeable and needs to assure a desired smoothness of the tampon, especially in the area of outwardly facing external surfaces of the ribs on the circumference of the tampon, and in addition to that, the material of the wrapper must be reliably attachable to the absorbent fibrous material as such. This means that, the material of the wrapper must be maintained in its position place on the absorbent fibrous band both during formation of the tampon, and also after the fibrous core material covered by said wrapper gets wet, and in particular also during subsequently removal of said tampon from a body cavity by means of the retaining string, which is connected to said fibrous material, so that the wrapper should not be separated from the fibrous material, which could in such a case lead to retaining of the wrapper within the body cavity. To this aim, in such tampons the material of said wrapper includes a determined amount of thermoplastic fibers, which are either suitable for binding with adhesives, or can as such also be melted and by increased temperature plasticized, so that are able to adhere and are also willing to amend the shape for the purpose of increasing smoothness of the tampon surface.

A tampon is described in WO 01/01910, the envelope of which is practically completely manufactured out of artificial materials. A tampon with such an envelope, when inserted into a body cavity, where the wrapper remains in a direct contact with the mucosa for several hours, may cause negative effects from the perspective of irritation and even inflammation of the mucosa. In addition, each tampon furnished with such a wrapper, regardless to the fact that the remainder of the tampon consist exclusively of biologically degradable natural fibers, presents a biologically non-degradable and therefore an environmentally unfriendly product, the recycling of which is generally not an option, wherein the decay of such artificial materials, for example in the process of natural composting, is exceptionally long-lasting and can last multiple decades.

Furthermore a tampon is published in EP 2 629 726 B1, the envelope of which is designed especially to ensure an improved sliding ability and a lower coefficient of friction on its external surface. Such a tampon is subsequently by means of thermal processing and applying pressure smoothed exclusively in the area of convex external surfaces of said ribs. For this purpose, the envelope comprises a mixture of fibers, a portion of which are thermoplastic fibers, wherein smoothing in said areas on the surface is carried out by means of heating the thermoplastic fibers to a temperature in the area of plasticization of the thermoplastic material of the fibers, while simultaneously ensuring a uniform gentle pressure towards the external surface of the envelope in the radial direction of the tampon. The envelope material is presented by a mixture of fibers from polyester (PES) and fibers from polyethylene (PE) of high density with a ratio of PE : PES = 36% : 66% to PE : PES = 50% : 50%, wherein the density of such two-component fibrous material is 12 to 20 g/m², preferably approximately 16 g/m², the envelope in the area of the convex external surfaces of said ribs is smoothed thanks to plasticization of polyethylene (PE) fibers at a temperature between 120°C and 160°C, and preferably between 122°C and 148°C, and at the same time thanks to subjecting plasticized fibers on the external surface of the envelope to a gentle and uniform pressure, which is with regards to the tampon applied radially inwards. Despite the improved sliding ability of the tampon on its surface, such a tampon due to unavoidable use of thermoplastic artificial materials still faces the problem of physiological acceptability in the case of a long-lasting direct contact with a human mucosa and at the same time also the problems in view of biological degradability and environment pollution.

A further aspect of using previously described state of the art tampons is connected with the shape of the tampon, on one hand in its dry state prior to use and on the other hand in its wet state after the use. The correctness if shape and the smoothness of the surface of the tampon in its dry state are characteristics, which influence the aesthetic look, which is important mainly for commercialization and non-problematic insertion of the tampon into each body cavity. In practice, the shape of the tampon in its wet state after use changes significantly. Namely, the fibers become wet during the use, which on one hand significantly, namely for the amount of absorbed liquid, increases the overall volume of the tampon, and on the other hand, due to each unequal distribution of fibers in a dry tampon, the shape of the tampon in its moisturized state will be considerably changed. Such unequal distribution of fibers inside the dry tampon, eventhough having a geometrically correct appearance from the outside, mainly results from the process of manufacturing the tampon as such. As mentioned, the tampon is manufactured of a fibrous band, which is cut to sections of a suitable length, each of them is initially folded and is upon that by means of rotation around the transversal axis, which presents the longitudinal axis of the manufactured tampon, by means of a fork-like tool, which is also rotated around said axis, helically wound into an approximately cylindrical shape, by which a blank is formed, which is then by means of suitable jaws on the circumference simultaneously and from multiple directions radially compressed into an approximately cylindrical tampon, on the circumference of which in the circumferential direction ribs are formed, which are distributed equidistantly with regards to each other and extend along the complete surface of the tampon. The overall appearance of such a tampon at least in its dry state is geometrically correctly, but distribution of fibers in its interior is obviously significantly unequal. One should therefore easily be clear that by wetting of such dry tampon, when as the liquid enters between the fibers, the mutual interaction between the previously compressed and therefore forcefully interwoven or otherwise interconnected fibers may become significantly different as soon as the tampon is wet. The fibers can namely change their shape and/or size during each wetting, and the presence of the liquid inside the tampon due to capillary effect and other phenomena significantly effects the mutual interaction of fibers during such wetting and in its saturated state when the tampon has absorbed the maximum possible amount of liquid with regard to each available amount of fibers. It should be pointed out that in most known tampons said fibrous band of pre-determined length is before winding into the shape of the blank and prior radial compression of the grooves simply cut in the area of both terminal edges, which means that the fibrous structure of the tampon also includes a straightforwardly cut-off terminal edge, which prior to said radial compression located on the external surface of said blank and which after said radial compression becomes practically invisible in the dry state of the tampon. Invisibility of said terminal edge is in most currently used tampons at least in its dry state still further supported by arrangement of said wrapper along the surface of the tampon, which then leads to correct shape and a required smoothness of the tampon surface. However, due to the presence of artificial fibers in classical wrappers, the use of such a wrapper is no longer an option in modern physiologically acceptable and biodegradable tampons.

Due to undesired application of the classic wrappers, which contain artificial fibers, in modern biodegradable physiologically acceptable and tampons serious problems may arise in connection with changing and maintaining of the tampon shape in the moisturized state thereof. Namely, in practice the problem arises due to modified interaction among fibers in their wet state, since by removing of the tampon from a body cavity, when the shape of the tampon is changed and because the tampon is maintained in contact with the internal surface of the body cavity, the layers of the tampon tend to peel relatively to each other, wherein the outermost layer of the fibrous band on the surface of the tampon, especially the cut-off terminal edge of said original fibrous band, is retained more intensive as the residual fibrous band layers, which are located deeply inside, which then leads to substantially helical unwinding of the fibrous band and the possibility of retaining of the torn area of the cut-off terminal area of the fibrous band although the tampon as such has been removed from the body cavity.

A still further tampon with spirally shaped grooves is disclosed in WO 02/076357 A2.

A relatively successful approach in resolving of said issues about changing the shape of a physiologically acceptable and biologically degradable tampon when transformed from its dry to wet state is described in the Slovenian patent application No SI-P-201900143, which is still unpublished, as well as in PCT/SI/2019/000008, to which the applicant refers by reference. Therein, it has been suggested that the free end portions of the fibrous band, prior to winding into a blank and subsequent radial compression, should not be cut-off straightforward and smoothly from the remaining endless fibrous band suitable for forming sections of at least approximately pre-determined length, but rather simply torn by means of a suitably designed tearing means. In such a case, prior to winding into a blank and prior to the subsequent radial compression, both terminal portions of each length section are much more smooth and gradual than after cutting-off by forming an usual straightforward edge, and consequently, the fibers in these areas are then suitably dragged apart and gradually diluted. Such an approach leads to the effect, that due to such gradual ending of the fibrous band, the shape of the wet band is much closer to the ideal cylindrical shape, and in addition to that, also said undesired effect of unwinding of a wet straightforwardly cut-off terminal area from a wet tampon is essentially reduced, which then also leads to elimination of helical unwinding of layers of fibrous material by removing the tampon from a body cavity. However, in the practice, by removing a tampon from a body cavity, despite to said gradual reduction of quantity of fibers in the area of torn-off terminal edges of the fibrous band there is still a problem with retaining of certain outwardly protruding and freely floating fibers, which may also be anyhow interwoven with neighboring fibers, which then retain the neighboring fibers, which may then by taking into account gradual thickening of fibers towards the interior of the tampon in the wet state of the last assist in retaining of still further fibers, so that even by the tampon with torn-off terminal edges of the fibrous band, the possibility of helically unwinding of fibrous layers in its wet state generally still persists and has actually never been completely resolved in any tampon as known in the prior art.

The present invention refers to a method for providing a shape retaining, physiologically acceptable and biologically degradable hygienic tampon, which is suitable for insertion into a body cavity of an user. Such a tampon generally consists of an absorbent fibrous core on the basis of suitably absorbent natural fibers, especially plant fibers on the basis of cotton and cellulose, which is obtained by radial compression of an at least approximately cylindrical blank. Said blank is formed of a predetermined section of an unwoven fibrous band having a predetermined fiber density per surface unit and a suitable length, width and thickness, depending on each desired weight and size of a dry tampon prior to the use thereof, wherein said length section is separated from an endless fibrous band by means of tearing, so that each terminal edge thereof is substantially uneven and in particular also gradually thinned. Said length section is subsequently, after insertion of a retaining string for removing each tampon after the use, helically wound around the geometric axis, which extends transversally with regard to the longitudinal axis of the fibrous band and is located within the plane of said fibrous band. The outer diameter of said helically wound blank of at least approximately cylindrical shape, which is formed of consisting of several fibrous layers placed one above the other, is substantially greater than the diameter of each manufactured tampon in its dry state. For the purpose of forming a final shape of the tampon, said blank is subjected to radial compression from several equidistant and symmetrically along its circumference distributed directions, by to which such obtained tampon is also furnished with a predetermined number of radial grooves, which are equidistantly spaced along the circumference of the tampon and can have equal or different depth with regard to each other and extend at least approximately parallel with each other and at least approximately in the longitudinal direction of the tampon, or are inclined at a predetermined angle with respect to the longitudinal geometric axis of the tampon, and wherein each of said ribs is furnished with a slightly deflected convex external surface and is located between each two neighboring grooves, so that the convex external surfaces of said ribs form an at least approximately cylindrical, or optionally a flattened cylindrical silhouette of each tampon. In addition, such tampon is optionally thinned on its one terminal area and is, for the purpose of easier insertion into a body cavity, furnished with a slightly rounded head, while on its opposite terminal it is furnished with a flexible retaining string, which is suitable for removing said tampon from each body cavity after the use.

The disclosure proposes that such tampon with previously stated characteristics is formed of a radially compressed blank consisting of several layers of a fibrous band, which are wound one above the other around said geometric axis, in which, prior to said radial compression of the blank, grooves are formed in the circumferential direction of said blank, which are in their profile shaped in form of letter V or U and each per se extend along the complete circumference thereof and are suitably spaced apart from each other. Said grooves are pressed into the layers at least to such extent, that the fibers of each upper fibrous band layer are pressed towards the fibers of each layer located below and are also interwoven there-with. Furthermore, said grooves are pressed by sliding of each exposed external fibrous band layer of each blank along a wedge-shaped smoothing tool, which is pressed towards the surface of the blank, which then leads to reduction of thickness of the fibrous band from the initial thickness in the unloaded state prior to helical winding to the final thickness, prior to said radial compression, wherein simultaneously each freely floating fibers in each terminal area on each external fibrous band layer are displaced from the surface of the blank towards the inside of each corresponding groove with the purpose of being interwoven with fibers located deeply therein, namely with fibers of the fibrous band layer, which is located below the top fibrous band layer.

The disclosure
further proposes that the number of said grooves along the length of the tampon should be at least 2 to 10. The depth of pressing of each groove into the torn fibrous band layer of final thickness, which is defined by the depth of impressing of said pressing and smoothing tool from the level of the external surface of the outer fibrous band layer in the direction towards the inner surface of the fibrous band and therefore also towards the fibrous band layer as located below it, should be at least 2/3 of said final thickness and preferably at least 3/4 of said final thickness of fibrous band layers, which are helically wound to an at least approximately cylindrical blank.

In fact, the invention proposes a process for manufacturing such shape retaining, physiologically acceptable and biologically degradable hygienic tampon with previously described characteristics, wherein said process generally comprises steps of
*i)* preparing a section of fibrous band consisting of unwoven and suitably absorbent natural fibers, in particular on the basis of cotton and cellulose, and having a suitable density, which is expressed in weight per surface unit, wherein said section of predetermined length is obtained by tearing-off from an endless fibrous band of predetermined width and thickness;
*ii)* placing a retaining string over said fibrous band;
*iii)* winding said fibrous band section around said transversal geometric axis in order to form a blank of a substantially cylindrical shape, the width of which approximately corresponds to the length of the tampon in its dry state and the diameter of which is essentially longer than the diameter of the manufactured tampon in its dry state;
*iv)* smoothing the surface of said blank during rotation thereof around its longitudinal axis for the purpose of achieving suitable orientation and interconnection of fibers and therefore strengthening said fibers on the outer surface of the blank, especially of freely floating fibers in the gradually thinned area within the torn-off terminal area of such helically wound fibrous band of the blank;
*v)* subjecting said blank to radial compression from several along the circumference equidistantly spaced directions, including forming of a front terminal area, namely a head of the tampon, which is intended for insertion into a body cavity, upon which the tampon is on its outer surface furnished with a certain number of radial grooves, which are equidistantly spaced, apart from each other and which have equal or different depth with respect to each other and extending at least approximately parallel with each other and at least approximately in the longitudinal direction of the tampon, or may alternatively extend inclined at a predetermined angle with respect to said longitudinal geometric axis of each tampon, wherein a rib with a slightly deflected convex external surface is available between each neighboring grooves, and wherein such convex external surfaces of said ribs all together form an at least approximately cylindrical, or optionally a flat cylindrical silhouette of each tampon. Moreover, such a tampon may optionally be thinned on its one terminal area and may for the purpose of easier insertion into each body cavity be ended with a slightly rounded head, while on its opposite terminal area it is furnished with a flexible retaining string, which is suitable for removing the tampon from a body cavity after the use thereof.

According to the invention, such process of manufacturing such a tampon is characterized in that in said step *iv)* after winding said section of fibrous band into a cylindrical blank, during rotation of said blank around said geometric axis, a predetermined number of grooves is formed in the circumferential direction of said blank, namely into the outer surface of each of one above the other placed fibrous band layers forming said blank, wherein said grooves are each per se shapet as letter V or U in profile and extend in circumferential direction and are suitably spaced apart from each other, wherein the depth of pressing said grooves into the layers is at least such, that the available fibers of each top layer are displaced in the direction towards each bottom layer as located each top layer, namely towards the fibers of said bottom layer inside of the groove and in such manner, that fibers of both layers become interwoven. In addition, during the same step *iv)* when forming said grooves, the friction between each wound fibrous band and a corresponding pressing i.e. smoothing tool, is at least such, that by winding the fibrous band as such is maintained in a suitably tensioned state and is at the same time on its surface smoothed in such manner, that the thickness of the fibrous band is reduced from the initial thickness in the unloaded state prior to winding to the final thickness of the wound fibrous band layers in the blank with impressed grooves, and at the same time at least the majority of freely floating fibers on each torn-off terminal area of the outmost fibrous band layer of the blank is directed and pressed towards the inside of said grooves in the longitudinal direction of said grooves in such manner, that said fibers are during the subsequent radial compression in step *v)* compressed deeply into the absorbent core of the tampon and interwoven with the residual fibers, which are present there-around.

According to the invention it is further foreseen, that in the step *iv)* at least 2 to 10 apart from each other spaced grooves are impressed simultaneously. In addition, it is foreseen that the grooves are pressed up to a predetermined depth, which is defined with the depth of impressing said pressing and smoothing tool from the level of the external surface of the outer fibrous band layer in the direction towards the inner surface of the fibrous band, namely towards the lower fibrous band layer within each blank, wherein said depth is at least 2/3 of said final thickness of the helically wound fibrous band layers in order to form an at least approximately cylindrical blank, and is preferably at least 3/4 of said final thickness of the helically wound fibrous band layers.

The invention will also be explained in some more detail with reference to accompanying drawings, in which
- Fig. 1: is a shape retaining, physiologically acceptable and biologically degradable hygienic tampon, which is obtainable by process according to the invention and which is schematically presented in isometric view;
- Fig. 2: is a blank consisting of wound fibrous material, which is a basis for manufacturing of a tampon according to Fig. 1 by means of radial compression, also presented schematically and in isometric view;
- Fig. 3: is a blank according to Fig. 2 in front view;
- Fig. 4: is a blank according to Fig. 2 in 3 in side view;
- Fig. 5: is a schematically presented cross-section of just three layers of fibrous material within the blank according to Figs. 2 - 4, namely along the diametrical plane of the blank;
- Fig. 6: is a conceptual presentation of a concept of forming each groove on the surface of the tampon blank according to the invention;
- Fig. 7: is a conceptual presentation of impressing a groove together with affecting relevant fibers within the exposed layers of the fibrous material of the tampon blank according to the invention;
- Fig. 8: is a general presentation of various tools, which are suitable for impressing said grooves into the surface of the blank; and
- Fig. 9: is a presentation of a simultaneous forming of multiple apart from each other spaced grooves.

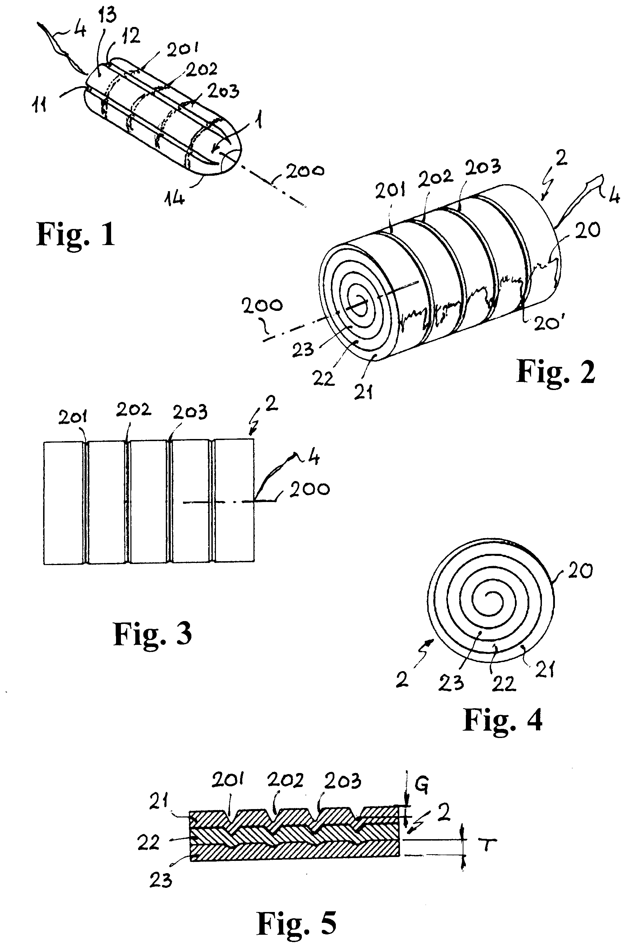

A shape retaining, physiologically acceptable and biologically degradable hygienic tampon 1, which is obtainable by process according to the invention, is as such shown in Fig. 1, and is suitable for insertion into a body cavity of an user. The tampon 1 consists of an absorbent fibrous core on the basis of suitably absorbent natural fibers, especially plant fibers on the basis of cotton and cellulose, and is formed by radial compression of an at least approximately cylindrical blank 2, which is as such shown on Figs. 2 - 4.

Said blank 2 is formed of a predetermined section of unwoven fibrous band having a predetermined fiber density per surface unit as well as a suitable length, width and thickness, depending on each weight and size of a dry tampon 1 prior to use, wherein said length section is separated from an endless fibrous band by means of tearing-off. The tampon 1 according to Fig. 1 is a digital tampon, which is generally available in different standardized sizes and is for the purpose of commercialization normally manufactured by using a 200 - 300 mm long and 30 - 60 mm wide unwoven fibrous band section with a specific density of 3,3 - 17,4 g/m. The advantage of tearing-off each length section of the fibrous material instead of straightforwardly cutting it from the remaining endless band of said width and specific fiber density as expressed in weight per surface unit, or length, is in that each terminal edge 20 of the fibrous band is substantially uneven and in particular also gradually thinned. Thus, the shape of the blank 2 (Fig. 4) can be much closer to the ideal cylindrical shape.

When forming the blank 2 as such, a retaining string 4 for removing each tampon 1 after the use is inserted into said blank 2, which is followed by helical winding of said blank 2 around the geometric axis 200, which extends transversally with regard to the longitudinal axis of the fibrous band and is located in the plane of the fibrous band. After said winding, the outer diameter of said helically wound blank 2. having at least approximately cylindrical shape and consisting of several fibrous layers 21, 22, 23 as placed one above the other, is substantially greater than the diameter of a manufactured tampon 1 in its dry state as such. The difference between the diameter of the blank 2 and the diameter of the manufactured tampon 1 is at least symbolically apparent in Figs. 1 and 2.

After said helical winding of the length section of fibrous band, said blank 2 is subjected to radial compression in several equidistantly and symmetrically along its circumference distributed directions, due to which then the tampon 1 as such (Fig. 1) is furnished with a predetermined number of radial grooves 11, 12, which are equidistantly spaced along the circumference of said tampon 1 and may have equal or different depth with regard to each other, extend at least approximately parallel with each other and either at least approximately in the longitudinal direction of the tampon 1 or in a direction inclined at a predetermined angle with respect to the longitudinal geometric axis 200 of the tampon 1, wherein each rib 13 with a slightly deflected convex external surface 130 is available between each two neighboring grooves 11, 12, and wherein such convex external surfaces 130 of said ribs 13 together form an at least approximately cylindrical or optionally a flattened cylindrical silhouette of each tampon 1. In additionally, such a tampon 1 is due to easier insertion into a body cavity optionally thinned in its one terminal area and is furnished with a slightly rounded head 14, while on its opposite terminal area it is furnished with a flexible retaining string 4, which is suitable for removing said tampon 1 from a body cavity after the use.

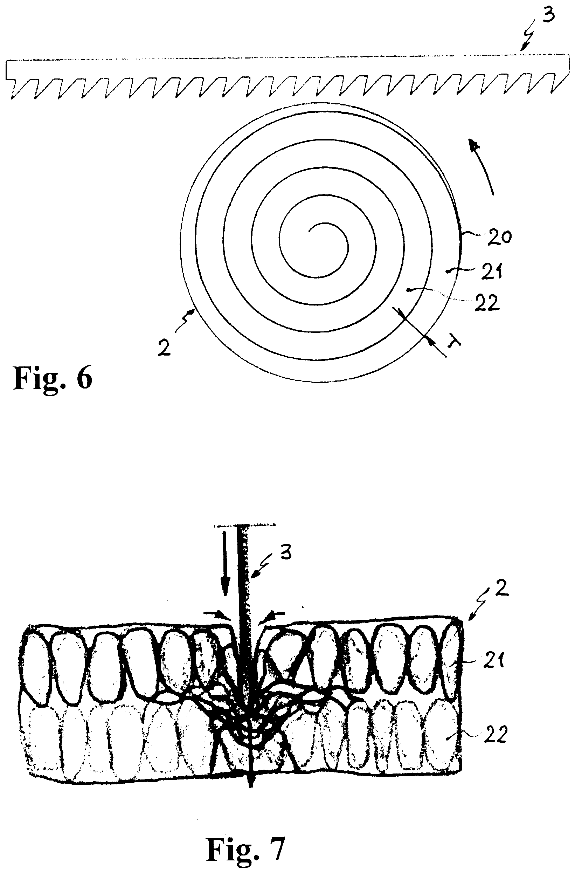

The tampon 1 is formed of a radially compressed blank 2, which includes several layers 21, 22, 23 of a fibrous band, which are wound one above the other around said axis 200, wherein said fibrous band layers 21, 22, 23, are placed one above the other (Figs. 2 - 4), such that prior to said radial compression of the blank 2, a plurality of grooves 201, 202, 203 is formed on said blank 2, which extend in the circumferential direction of the blank 2 and are suitably spaced apart from each other each, wherein each of them is in profile shaped in form of letter V or U and extends continuously along the complete circumference of the blank 2. Said grooves 201, 202, 203 are impressed into the layers 21, 22, 23 at least to such extent, that the fibers of each upper fibrous band layer 21 are impressed towards the fibers of the layer 22 which is located below said upper layer 21 (Fig. 7) and are then interwoven there-with.

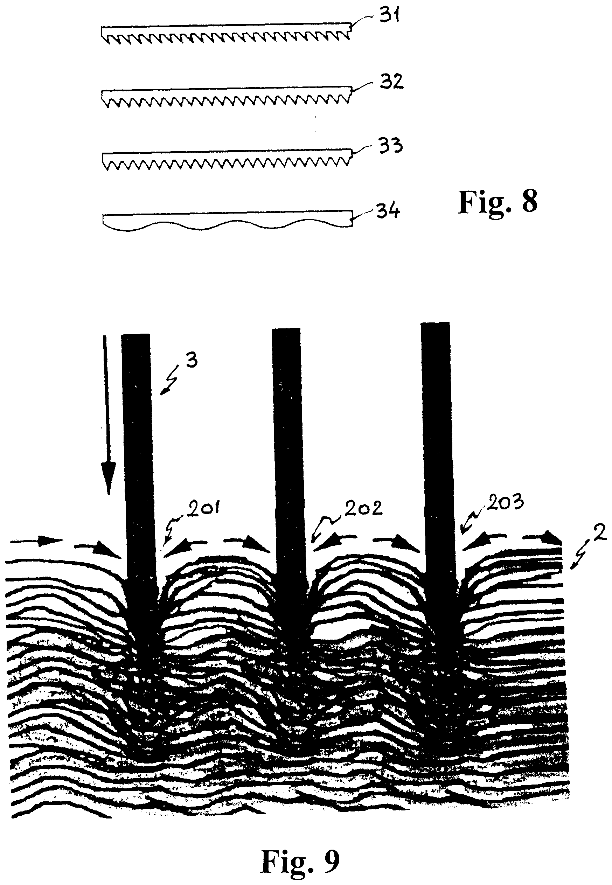

Furthermore, said grooves 201, 202, 203 are impressed into the fibrous band layers 21, 22, 23 during sliding of each exposed outer fibrous band layer 21 of each blank 2 along a wedge-shaped smoothing tool 3; 31, 32, 33, 34, which is pressed towards the surface of the blank 2. Said tool 3 and the blank 2 are presented in Fig. 6 s in a state, where the blank 2 is separated from the tool 3; 31, 32, 33, 34, while the interaction between the tool 3; 31, 32, 33, 34 and the blank 2 is shown in Figs. 7 and 9. In addition various possible embodiments 31, 32, 33, 34 of said tool 3 are shown in Fig. 8, which are each per se suitable for impressing said grooves 201, 202, 203. In the view of the invention as such, it is merely important that the transversal cross section of such tool 3; 31, 32, 33, 34 is properly shaped in profile, namely in the shape of letter V or U, which then determines the profile of each impressed groove 201, 202, 203, but it is also important, that it comprises one or more inclinations, in the area of which the tool 3 presses the fibers in the radial direction of the blank 2 towards the interior thereof simultaneously with rotating said blank 2 in the direction of the arrow according to Fig. 6. Due to said impression of the grooves 201, 202, 203 and the friction, namely a resistance caused by it, during said rotation of the blank 2 the layer is additionally extended, which then leads to reduction of thickness of the fibrous band from the initial thickness in the unloaded state prior to helical winding, to the final thickness T, prior to radial compression. At the same time, each freely floating fibers 20' in the terminal area 20 of the outer fiber band layer 21, which could otherwise in the wet state of the tampon and especially during its removal from each body cavity by means of said retaining the string 4 initiate undesired peeling of the layer 21, are herewith displaced from the surface of the blank 2 towards the interior of each corresponding groove 201, 202, 203 with the possibility of being interwoven with neighboring fibers located deeply therein, namely with fibers of the fibrous band layer 22, which is located below the top fibrous band layer 21.

The number of said grooves 201, 202, 203 along the length of the tampon 1 should be at least 2 to 10. The tampon 1 according to Fig. 1, which is made of the blank 2 according to Figs. 2 - 4, is exclusively for illustrative purposes furnished with four grooves 201, 202, 203, which however does not present any limitation with regard to the scope of protection of the invention.

In the view of effectiveness of the invention itself it is meaningful that the depth G of impressing of each groove 201, 202, 203 into the torn fibrous band layer (21) of final thickness T, which is defined with the depth of pressing said pressing and smoothing tool 3; 31, 32, 33, 34 from the level of the external surface of the outer fibrous band layer 21 in the direction towards the inner surface of the fibrous band, namely towards the lower fibrous band layer 22, is at least 2/3 of said final thickness T of fibrous band layers 21, 22, 23, which are helically wound in order to form an at least approximately cylindrical blank 2. It is especially efficient, if the depth G of impressing of each groove 201, 202, 203 into said layer 21 is at least 3/4 of said final thickness T of the fibrous band layers 21, 22, 23, which are helically wound into an at least approximately cylindrical blank 2.

The invention proposes a process for manufacturing of such shape retaining, physiologically acceptable and biologically degradable hygienic tampon 1 with previously described characteristics. The process of manufacturing said tampon 1 generally comprises steps of
*i)* preparing a section of a fibrous band consisting of unwoven and suitably absorbent natural fibers, in particular on the basis of cotton and cellulose, and having a suitable density, which is determined by weight per surface unit, wherein said section of predetermined length is obtained by tearing-off from an endless fibrous band of predetermined width and thickness;
*ii)* placing a retaining string 4 over said fibrous band;
*iii)* winding said fibrous band section around the transversal geometric axis 200 in order to form the blank 2 of substantially cylindrical shape, the width of which at least approximately corresponds to the length of the tampon 1 in its dry state and the diameter of which is essentially longer than the diameter of the manufactured tampon 1 in its dry state;
*iv)* smoothing the surface of the blank 2 during rotation thereof around its longitudinal axis 200 for the purpose of achieving suitable orientation and interconnection of fibers and therefore strengthening said fibers on the external surface of the blank 2, especially of freely floating fibers 20' in the gradually thinned area in the torn-off terminal area 20 of such helically wound fibrous band of the blank 2;
*v)* subjecting said blank 2 to radial compression from several along the circumference equidistantly spaced directions, including forming of a front terminal area, namely a head 14 of the tampon 1, which is suitable for insertion into a body cavity, upon which the tampon 1 is on its external surface furnished with certain number of radial grooves 11, 12, which are equidistantly spaced apart from each other and may have either equal or different depth with respect to each other, and which are extending at least approximately parallel with each other and either at least approximately in the longitudinal direction of the tampon 1 or in a direction, which is inclined at a predetermined angle with regard to the longitudinal geometric axis 200 of each tampon 1, wherein each of said ribs 13 comprises a slightly deflected convex external surface 130 and is located between two neighboring grooves 11, 12, so that the convex external surfaces 130 of said ribs 13 together form an at least approximately cylindrical, or optionally a flat cylindrical, silhouette of each tampon 1, while in addition to that, such tampon 1 is optionally on its one terminal area thinned and is due to easier insertion into corresponding body cavity ended with a slightly rounded head 14, and is on its opposite terminal area furnished with a flexible retaining string 4, which is suitable for removing each tampon 1 from said body cavity after the use thereof.

The process according to the invention is characterized in that in said step *iv),* namely after winding said section of fibrous band due to formation of a cylindrical blank 2, and during rotation of said blank 2 around said geometric axis 200, a predetermined number of continuous grooves 201, 202, 203 is formed into the external surface of each of one above the other arranged fibrous band layers 21, 22, 23 of said blank 2, which are in profile V or U letter shaped and extend in circumferential direction at suitable distances apart from each other. The depth G (Fig. 5) of impressing said grooves 201, 202, 203 is at least such that in the area of each of said grooves 201, 202, 203 all disposable fibers on each top layer 21 are displaced inwardly in a direction towards the layer 22, which is located below said top layer 21, namely towards the fibers of said bottom layer 22 within the groove 201, 202, 203, namely in such manner, that fibers of both layers become interwoven (Figs. 7 and 9).

In addition, by forming of said grooves 201, 202, 203 in the same step *iv)* the friction between each wound fibrous band and the pressing, i.e. smoothing tool 3; 31, 32, 33, 34 should be at least such, that the fibrous band is during said winding maintained in a suitably tensioned state and is at the same time on its surface smoothed in such manner, that the thickness of the fibrous band is reduced from an initial thickness in the unloaded state prior to winding and subsequent impression of the grooves 201, 202, 203, to the final thickness T of the wound fibrous band layers 21, 22, 23.

At the same time at least the majority of freely floating fibers 20' on each torn-off terminal area 20 on each external fibrous band layer 21 of the blank 2 is directed and impressed towards the inside of said grooves 201, 202, 203 in the longitudinal direction of said grooves 201, 202, 203 in such manner, that said fibers are during said radial compression in step *iv)* impressed deeply into the absorbent core of the tampon 1 and there interwoven with the residual fibers, which are present there.

According to the invention it is further foreseen that in the step *iv)* at least 2 to 10 apart from each other spaced grooves 201, 202, 203 are simultaneously impressed. In addition, it is foreseen that said grooves 201, 202, 203 are impressed up to a predetermined depth G, which is defined by the depth of impressing said pressing and smoothing tool 3; 31, 32, 33, 34 from the level of the external surface of the outer fibrous band layer 21 in the direction towards the inner surface of the fibrous band, namely towards the lower fibrous band layer 22 within the blank 2, wherein said depth G should be at least 2/3 of said final thickness T, preferably at least 3/4 of said final thickness T of each of the helically wound fibrous band layers 21, 22, 23 within said at least approximately cylindrical blank 2.

It should be clear to every person skilled in the art that during such manufacturing of the blank 2, said freely floating fibers 20' on the torn-off terminal area 20 of the fibrous band are at least substantially directed inwardly into of the grooves 201, 202, 203, in the area of which said fibers are then interwoven with the fibers as available therein, and moreover, the fibers are also interwoven with each other and thanks to the groove profile in the form of letter V or U, the fibers of each external layer 21 and each layer 22 below said external layer 21 are then also jammed between each other. Such behavior merely occurs in the longitudinal direction of the blank 2, namely parallel to the axis 200, however in the next step v) during said compression of the blank 2 in its radial direction towards the inside of the blank 2, namely in the perpendicular direction with regards to the axis 200, the fibers are interwoven amongst themselves again and are therefore stabilized in such manner, that even in a wet state the undesired peeling of layers 21, 22, 23 cannot occur.

## Claims

1. Process for manufacturing a shape retaining, physiologically acceptable and biologically degradable hygienic tampon (1), generally comprising steps of
*i)* preparing a section of fibrous band from unwoven and suitably absorbent natural fibers, in particular on the basis of cotton and cellulose, and having a suitable density, which is expressed in weight per surface unit, wherein said section of predetermined length is obtained by tearing-off from an endless unwoven fibrous band of predetermined width and thickness;
*ii)* placement of a retaining string (4) over said fibrous band;
*iii)* winding said fibrous band section around a geometric axis (200) in order to form a substantially cylindrical blank (2), the width of which approximately corresponds to the length of the tampon (1) in its dry state, while its diameter is essentially longer than the diameter of the manufactured tampon (1) in its dry state;
*iv)* smoothing the external surface of the blank (2) during rotation thereof around its longitudinal axis (200) for the purpose of achieving suitable orientation and interconnection of fibers and therefore strengthening of fibers on the external surface of the blank (2), especially of freely floating fibers (20') in the gradually thinned area within the torn-of terminal area (20) of such helically wound fibrous band of the blank (2);
*v)* subjecting said blank (2) to radial compression from several along the circumference symmetrically and equidistantly spaced directions, including forming of a front terminal area, namely a head (14) of the tampon (1), which is suitable for insertion into a body cavity, upon which the tampon (1) is on its external surface furnished with a certain number of radial grooves (11, 12), which are equidistantly spaced apart from each other and have either equal or different depth with respect to each other, and which are extending at least approximately parallel with each other and either at least approximately in the longitudinal direction of the tampon (1) or in a direction, which is inclined at a predetermined angle according to the longitudinal geometric axis (200) of such tampon (1),
wherein each rib (13), which is located between two neighboring grooves (11, 12), is furnished with a slightly deflected convex external surface (130), so that such convex external surfaces (130) of all disposable ribs (13) together form an at least approximately cylindrical, or optionally a flat cylindrical, silhouette of the tampon (1), wherein such tampon (1) is on its one terminal area optionally thinned and is due to easier insertion into a body cavity ended with a slightly rounded head (14), while in the opposite terminal area it is furnished with a flexible retaining string (4), which is suitable for removing such tampon (1) from a body cavity after the use thereof, **characterized in that**
in step *iv)* upon winding of said section of fibrous band into a cylindrical blank (2), during rotation of said blank (2) around said geometric axis (200),
a pre-determined number of suitably apart from each other spaced continuous circumferential grooves (201, 202, 203) in form of letter V or U in profile is formed on said blank (2) on the external surface of each of the fibrous band layers (21, 22, 23), which are placed one above the other in order to form said blank (2), wherein the depth (G) of impressing said grooves (201, 202, 203) into said layers (21, 22, 23) is at least such that the available fibers on each top layer (21) in the area within the groove (201, 202, 203), are displaced inwardly in a direction towards the layer (22) which is located underneath said top layer (21), namely towards the fibers of each bottom layer (22) namely in such manner, that fibers of both layers are interwoven, **and in that**
- in the same step *iv)* during forming of said grooves (201, 202, 203) the friction between each wound fibrous band and the pressing, i.e. smoothing tool (3; 31, 32, 33, 34) is at least such, that by winding the fibrous band is maintained in a suitably tensioned state and is at the same time on its surface smoothed in such manner, that the thickness of the fibrous band is reduced from the initial thickness in the unloaded state prior to winding to the final thickness (T) of the wound fibrous band layers (21, 22, 23) in the blank (2) with impressed grooves (201, 202, 203), and at the same time at least the majority of the freely floating fibers (20') in each torn-of terminal area (20) of each external fibrous band layer (21) of the blank (2) is directed inwardly and pressed towards the interior of said grooves (201, 202, 203) in the longitudinal direction of said grooves (201, 202, 203) in such manner, that said fibers are during said radial compression in step *iv)* impressed deeply into the absorbent core of the tampon (1) and interwoven with those disposable fibers, which are present there.

2. Process according to Claim 1, **characterized in that,** in the step *iv)* at least 2 to 10 apart from each other spaced grooves (201, 202, 203) are simultaneously impressed.

3. Process according to Claims 1 and 2, **characterized in that** said grooves (201, 202, 203) are impressed up to a predetermined depth (G), which is defined by the depth of impressing of said pressing and smoothing tool (3; 31, 32, 33, 34) from the level of the external surface of the outer fibrous band layer (21) in a direction towards the inner surface of the fibrous band, namely towards the lower fibrous band layer (22) of each blank (2), wherein said depth (G) is at least 2/3 of said final thickness (T) of the helically wound fibrous band layers (21, 22, 23) in an at least approximately cylindrical blank (2).

4. Process according to Claim 3, **characterized in that,** each groove (201, 202, 203) is impressed into the layer (21) up to the depth (G), which is at least 3/4 of said final thickness (T) of the helically wound fibrous band layers (21, 22, 23) in an at least approximately cylindrical blank (2).

## Patentansprüche

1. Verfahren zur Herstellung eines formstabilen, physiologisch verträglichen und biologisch abbaubaren Hygienetampons (1), welches im Allgemeinen die folgenden Schritte umfasst:
*i*. Herstellen eines Abschnitts eines Faserbandes aus ungewebten und ausreichend saugfähigen Naturfasern, insbesondere auf der Basis von Baumwolle und Zellulose, und von einer geeigneten Dichte, die in Gewicht pro Flächeneinheit ausgedrückt wird, wobei der Abschnitt mit vorbestimmter Länge durch Abreißen von einem endlosen ungewebten Faserband mit vorbestimmter Breite und Dicke erhalten wird;
*ii.* Anordnen einer Rückhalteschnur (4) über dem Faserband;
*iii.* Aufwickeln des Faserbandabschnitts um eine geometrische Achse (200), um einen im Wesentlichen zylindrischen Rohling (2) zu bilden, dessen Breite ungefähr der Länge des Tampons (1) in seinem trockenen Zustand entspricht, während sein Durchmesser wesentlich länger ist als der Durchmesser des hergestellten Tampons (1) in seinem trockenen Zustand;
*iv.* Glätten der Außenfläche des Rohlings (2) während seiner Drehung um seine Längsachse (200), um eine geeignete Ausrichtung und Verbindung der Fasern und somit eine Verstärkung der Fasern auf der Außenfläche des Rohlings (2) zu erreichen, insbesondere der frei schwebenden Fasern (20') in dem allmählich verdünnten Bereich innerhalb des abgerissenen Endbereichs (20) eines solchen spiralförmig gewundenen Faserbands des Rohlings (2);
v. Unterziehen des Rohlings (2) einer radialen Kompression aus mehreren entlang des Umfangs symmetrisch und äquidistant beabstandeten Richtungen, einschließlich Bilden eines vorderen Endbereichs, nämlich eines Kopfes (14) des Tampons (1), der zum Einführen in eine Körperhöhle geeignet ist, wobei der Tampon (1) auf seiner Außenfläche mit einer bestimmten Anzahl von radialen Nuten (11, 12) versehen ist, die in gleichem Abstand voneinander angeordnet sind und entweder gleiche oder unterschiedliche Tiefe in Bezug aufeinander aufweisen, und die sich zumindest annähernd parallel zueinander und entweder zumindest annähernd in Längsrichtung des Tampons (1) oder in einer Richtung erstrecken, die in einem vorbestimmten Winkel zur geometrischen Längsachse (200) eines solchen Tampons (1) geneigt ist,
wobei jede Rippe (13), die sich zwischen zwei benachbarten Nuten (11, 12) befindet, mit einer leicht gebogenen konvexen Außenfläche (130) versehen ist, so dass diese konvexen Außenflächen (130) aller verfügbaren Rippen (13) zusammen eine zumindest annähernd zylindrische oder wahlweise eine flache zylindrische Silhouette des Tampons (1) bilden, wobei ein solcher Tampon (1) an seinem einen Endbereich optional verdünnt ist und zum leichteren Einführen in eine Körperhöhle mit einem leicht abgerundeten Kopf (14) endet, während er im entgegengesetzten Endbereich mit einem flexiblen Rückholfaden (4) versehen ist, der zum Entfernen eines solchen Tampons (1) aus einer Körperhöhle nach dessen Verwendung geeignet ist, **dadurch gekennzeichnet, dass**
in Schritt *iv)* beim Aufwickeln des Abschnitts des Faserbandes zu einem zylindrischen Rohling (2) während der Drehung des Rohlings (2) um die geometrische Achse (200)
eine vorbestimmte Anzahl von in geeignetem Abstand voneinander angeordneten, durchgehenden Umfangsnuten (201, 202, 203) in Form des Buchstabens V oder U im Profil auf dem Rohling (2) auf der Außenfläche jeder der Faserbandschichten (21, 22, 23) gebildet sind, die übereinander angeordnet sind, um den Rohling (2) zu bilden,
wobei die Tiefe (G) des Eindrückens der Nuten (201, 202, 203) in die Schichten (21, 22, 23) mindestens so groß ist, dass die verfügbaren Fasern auf jeder oberen Schicht (21) in dem Bereich innerhalb der Nut (201, 202, 203) nach innen in Richtung zu der Schicht (22) verschoben werden, die sich unter der oberen Schicht (21) befindet, nämlich zu den Fasern jeder unteren Schicht (22),
und zwar auf eine solche Weise, dass die Fasern beider Schichten miteinander verwoben werden, **und dass**
- im gleichen Schritt *iv)* während des Ausbildens der Nuten (201, 202, 203) die Reibung zwischen jedem aufgewickelten Faserband und dem Press- bzw. Glättwerkzeug (3; 31, 32, 33, 34) mindestens so ist, dass das Faserband durch das Aufwickeln in einem geeignet gespannten Zustand gehalten und gleichzeitig auf seiner Oberfläche so geglättet wird, dass die Dicke des Faserbandes von der Anfangsdicke im unbelasteten Zustand vor dem Aufwickeln auf die Enddicke (T) der aufgewickelten Faserbandschichten (21, 22, 23) im Rohling (2) mit eingedrückten Nuten (201, 202, 203) reduziert wird und gleichzeitig zumindest der Großteil der frei schwebenden Fasern (20') in jedem abgerissenen Endbereich (20) jeder äußeren Faserbandschicht (21) des Rohlings (2) in der Längsrichtung der Nuten (201, 202, 203) derart nach innen gerichtet und in Richtung auf das Innere der Nuten (201, 202, 203) gedrückt wird, dass die Fasern während der radialen Kompression in Schritt *iv)* tief in den absorbierenden Kern des Tampons (1) eingedrückt und mit den dort vorhandenen Einwegfasern verwoben werden.

2. Verfahren nach Anspruch 1, **dadurch gekennzeichnet, dass** in Schritt *iv)* mindestens 2 bis 10 voneinander beabstandete Nuten (201, 202, 203) gleichzeitig eingedrückt werden.

3. Verfahren nach Anspruch 1 und 2, **dadurch gekennzeichnet, dass** die Nuten (201, 202, 203) bis zu einer vorbestimmten Tiefe (G) eingedrückt werden, die durch die Eindrücktiefe des Press- und Glättwerkzeugs (3; 31, 32, 33, 34) ausgehend von der Ebene der Außenfläche der äußeren Faserbandschicht (21) in eine Richtung zur Innenfläche des Faserbandes, nämlich in Richtung zur unteren Faserbandschicht (22) jedes Rohlings (2), definiert ist, wobei die Tiefe (G) mindestens 2/3 der Enddicke (T) der schraubenförmig gewickelten Faserbandschichten (21, 22, 23) in einem zumindest annähernd zylindrischen Rohling (2) beträgt.

4. Verfahren nach Anspruch 3, **dadurch gekennzeichnet, dass** jede Nut (201, 202, 203) in die Schicht (21) bis zu der Tiefe (G) eingedrückt wird, die mindestens 3/4 der Enddicke (T) der schraubenförmig gewundenen Faserbandschichten (21, 22, 23) in einem zumindest annähernd zylindrischen Rohling (2) beträgt.

## Revendications

1. Procédé de fabrication d'un tampon hygiénique (1) à maintien de forme, physiologiquement acceptable et biologiquement dégradable, comprenant généralement les étapes de
*i)* préparer une section de bande fibreuse à partir de fibres naturelles non tissées et convenablement absorbantes, en particulier à base de coton et de cellulose, et ayant une densité appropriée, qui est exprimée en poids par unité de surface, dans lequel ladite section de longueur prédéterminée est obtenue par arrachage d'une bande fibreuse non tissée sans fin de largeur et d'épaisseur prédéterminées ;
*ii)* placer un cordon de maintien (4) sur ladite bande fibreuse ;
*iii)* enrouler ladite section de bande fibreuse autour d'un axe (200) géométrique afin de former une ébauche (2) sensiblement cylindrique, dont la largeur correspond approximativement à la longueur du tampon (1) à l'état sec, tandis que son diamètre est sensiblement plus long que le diamètre du tampon (1) fabriqué à l'état sec ;
*iv)* lisser la surface externe de l'ébauche (2) pendant sa rotation autour de son axe (200) longitudinal dans le but d'obtenir une orientation et une interconnexion appropriées des fibres et par conséquent un renforcement des fibres sur la surface externe de l'ébauche (2), en particulier des fibres flottant librement (20') dans la zone progressivement amincie à l'intérieur de la zone terminale déchirée (20) de cette bande fibreuse enroulée en hélice de l'ébauche (2) ;
*v)* soumettre ladite ébauche (2) à une compression radiale depuis plusieurs directions espacées de manière symétrique et équidistante le long de la circonférence, comprenant la formation d'une zone terminale avant, à savoir une tête (14) du tampon (1), qui est appropriée pour une insertion dans une cavité corporelle, sur laquelle le tampon (1) est muni sur sa surface externe d'un certain nombre de rainures (11, 12) radiales, qui sont espacées de manière équidistante les unes des autres et ont une profondeur égale ou différente les unes par rapport aux autres, et qui s'étendent au moins approximativement parallèlement les unes aux autres et soit au moins approximativement dans la direction longitudinale du tampon (1) soit dans une direction, qui est inclinée à un angle prédéterminé selon l'axe (200) géométrique longitudinal de ce tampon (1),
dans lequel chaque nervure (13), qui est située entre deux rainures (11, 12) voisines, est dotée d'une surface externe convexe (130) légèrement fléchie, de sorte que ces surfaces externes convexes (130) de toutes les nervures (13) disponibles forment conjointement une silhouette au moins approximativement cylindrique, ou éventuellement cylindrique plate, du tampon (1), dans lequel un tel tampon (1) est éventuellement aminci sur sa zone terminale et est en raison d'une insertion plus facile dans une cavité corporelle terminé par une tête (14) légèrement arrondie, tandis que dans la zone terminale opposée il est muni d'un cordon de retenue (4) flexible, qui est approprié pour retirer un tel tampon (1) d'une cavité corporelle après son utilisation, **caractérisé en ce que**
à l'étape *iv)* lors de l'enroulement de ladite section de bande fibreuse en une ébauche (2) cylindrique, pendant la rotation de ladite ébauche (2) autour dudit axe (200) géométrique,
un nombre prédéterminé de rainures (201, 202, 203) circonférentielles continues convenablement espacées les unes des autres, à profil en forme de lettre V ou U sont formées sur ladite ébauche (2) sur la surface externe de chacune des couches de bande fibreuse (21, 22, 23), qui sont placées les unes au-dessus des autres afin de former ladite ébauche (2),
dans lequel la profondeur (G) d'impression desdites rainures (201, 202, 203) dans lesdites couches (21, 22, 23) est au moins telle que les fibres disponibles sur chaque couche (21) supérieure dans la zone à l'intérieur de la rainure (201, 202, 203), sont déplacées vers l'intérieur dans une direction vers la couche (22) qui est située sous ladite couche (21) supérieure, à savoir vers les fibres de chaque couche (22) inférieure à savoir de telle manière que les fibres des deux couches sont entrelacées, **et en ce que**
- à la même étape *iv)* lors de la formation desdites rainures (201, 202, 203) le frottement entre chaque bande fibreuse enroulée et l'outil (3 ; 31, 32, 33, 34) de pressage, c'est-à-dire de lissage, est au moins tel que, par enroulement, la bande fibreuse est maintenue dans un état convenablement tendu et est en même temps lissée sur sa surface de telle manière que l'épaisseur de la bande fibreuse est réduite de l'épaisseur initiale dans l'état non chargé avant l'enroulement jusqu'à l'épaisseur finale (T) des couches de bande fibreuse (21, 22, 23) enroulées dans l'ébauche (2) avec des rainures (201, 202, 203) imprimées, et en même temps au moins la majorité des fibres flottant librement (20') dans chaque zone terminale déchirée (20) de chaque couche de bande fibreuse (21) externe de l'ébauche (2) est dirigée vers l'intérieur et pressée vers l'intérieur desdites rainures (201, 202, 203) dans la direction longitudinale desdites rainures (201, 202, 203) de telle manière que lesdites fibres sont pendant ladite compression radiale de l'étape *iv)* imprimées profondément dans le noyau absorbant du tampon (1) et entrelacées avec les fibres disponibles qui y sont présentes.

2. Procédé selon la revendication 1, **caractérisé en ce que,** à l'étape *iv),* au moins 2 à 10 rainures (201, 202, 203) espacées les unes des autres sont imprimées simultanément.

3. Procédé selon les revendications 1 et 2, **caractérisé en ce que** lesdites rainures (201, 202, 203) sont imprimées jusqu'à une profondeur (G) prédéterminée, qui est définie par la profondeur d'impression dudit outil (3 ; 31, 32, 33, 34) de pressage et de lissage à partir du niveau de la surface externe de la couche de bande fibreuse (21) externe dans une direction vers la surface interne de la bande fibreuse, à savoir vers la couche de bande fibreuse (22) inférieure de chaque ébauche (2), dans lequel ladite profondeur (G) est au moins égale aux 2/3 de ladite épaisseur finale (T) des couches de bande fibreuse enroulées en hélice (21, 22, 23) dans une ébauche (2) au moins approximativement cylindrique.

4. Procédé selon la revendication 3, **caractérisé en ce que** chaque rainure (201, 202, 203) est imprimée dans la couche (21) jusqu'à la profondeur (G) qui est au moins égale aux 3/4 de ladite épaisseur finale (T) des couches de bande fibreuse (21, 22, 23) enroulées en hélice dans une ébauche (2) au moins approximativement cylindrique.
